# EUROPEAN PATENT APPLICATION

(11) **EP 0 925 769 A2**
(43) Date of publication of application: **30.06.1999**
(21) Application number: 98310340.9
(22) Date of filing: 16.12.1998
(51) Int. Cl.: A61F 13/42

(54) **Disposable diaper**

(30) Priority: 16.12.1997 JP 34623897
(71) Applicant: UNI-CHARM CORPORATION, Kawanoe-shi Ehime-ken (JP)
(72) Inventor: Shimoe, Nariaki, c/o Research & Development Div., Mitoyo-gun, Kagawa-ken 769-1602 (JP); Otsubo, Toshifumi, c/o Research & Development Div., Mitoyo-gun, Kagawa-ken 769-1602 (JP)
(74) Representative: Parry, Christopher Stephen

(57) **Abstract**

A disposable diaper including a topsheet, a backsheet and a core disposed therebetween so as to define a front waist region, a rear waist region and a crotch region extending therebetween, the backsheet being formed at least in a transversely middle location of the crotch region with a transparent zone through which a wetness of the core due to urine can be observed.

## Description

The present invention relates to a disposable diaper for absorption and containment of excretion.

Disposable diapers have usually employed, as its backsheet, a sheet presenting a relatively low light-transmission and/or a relatively high light-diffusion and being opaque to a degree that its absorbent core can not substantially be seen therethrough. Such sheet has contributed to conceal the core soiled with excretion.

Japanese Patent Application Disclosure Gazette (Kokai) No. Hei9-140742 describes such a disposable diaper characterized by that there is provided between the backsheet and the core with an indicator adapted to become visible through the backsheet when the indicator is wetted with urine. This indicator exhibits a striking color when wetted and, therefore, the indicator is secured to an inner surface of the backsheet by means of hot melt adhesive so that the striking color may be readily identified through the backsheet. By utilizing such indicator, a proper timing for exchanging the soiled diaper with a fresh diaper can be reliably signaled to a user.

According to this prior art, the indicator is made of a printed hydrophilic sheet and, for this printing, a particular type of ink is selected, which is harmless and free from any sanitary risque even if such ink comes in contact with the skin or the mouth of a baby wearing the diaper. However, chemical composition of used ink as well as sensibility of the baby wearing the diaper are not always uniform. Problems of sanitary safety possibly occurring due to use of ink will be avoided if urination can be visually detected externally of the diaper without relying upon use of ink.

It is therefore an object of the present invention to provide a disposable diaper satisfying such a demand.

According to the invention, there is provided a disposable diaper comprising a liquid-permeable topsheet, a liquid-impermeable backsheet and a liquid-absorbent core disposed therebetween so as to define a front waist region, a rear waist region and a crotch region extending therebetween wherein: the backsheet includes a transparent zone occupying at least a transversely middle location of the crotch region and allowing a wetness of the core to be observed and an opaque zone extending around the transparent zone and through which the core can not substantially be visible.

Preferably, the backsheet is opaque in the remainder except the transparent zone.

Preferably, the transparent zone extends from the crotch region into said front waist region.

Preferably, a surface of the core facing the transparent zone is formed with an indicator adapted to become visible when wetted with urine.

Preferably, the backsheet inclusive of the transparent zone is entirely formed by a plastic sheet.

Preferably, the transparent zone is formed on the front side of the diaper at its transversely middle location.
Fig. 1 is a perspective view showing an embodiment of a disposable diaper according to the present invention as partially broken away; and
Fig. 2 is a plan view showing another embodiment of the disposable diaper according to the present invention.

Details of a disposable diaper according to the invention will be more fully understood from the description given hereunder with reference to the accompanying drawings.

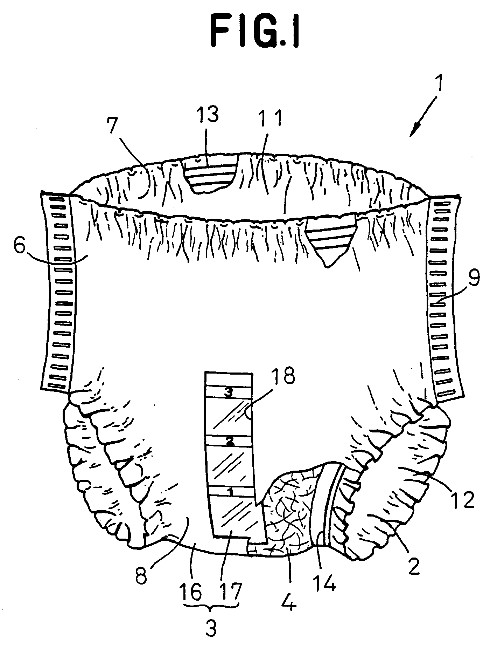

Disposable diaper 1 shown by Fig. 1 in a perspective view as partially broken away is of pull-on or shorts type. The diaper 1 comprises a liquid-permeable topsheet 2, a liquid-impermeable backsheet 3 and a liquid-absorbent core 4 disposed between these two sheets 2, 3. These sheets 2, 3 and core 3 defines a front waist region 6, a rear waist region 7 and a crotch region 8 extending between these two trunk regions 6, 7. The topsheet 2 and the backsheet 3 extend outward beyond a peripheral edge of the core 4 and are joined together in these extensions. The front and rear waist regions 6, 7 are placed one upon another along their respective transversely opposite side edges and joined together at spots 9 intermittently arranged along these side edges so as to form a waist-opening 11 and a pair of leg-openings 12. These openings 11, 12 are provided adjacent their peripheral edges with elastic members 13, 14, respectively, so as to extend circumferentially of the respective openings 11, 12. These elastic members 13, 14 are disposed between the topsheet 2 and the backsheet 3 and secured to an inner surface of at least one of the topsheet 2 and the backsheet 3.

The topsheet 2 is made of a nonwoven fabric or an apertured plastic sheet and may be substantially opaque to a degree preventing the core 4 from being seen therethrough, or may be translucent to a degree allowing the core 4 to be more or less seen therethrough or may be substantially transparent to a degree allowing the core 4 to be readily seen therethrough.

The backsheet 3 comprises a first plastic sheet 16 which is substantially opaque to a degree preventing the core 4 from being seen therethrough and a second plastic sheet 17 which is substantially transparent to a degree allowing the core 4 to be readily seen therethrough. The first plastic sheet 16 substantially covering the front and rear waist regions 6, 7 and the crotch region 8 is partially cut out at least in a transversely middle and longitudinally front zone in the proximity of a wearer's excretive organ so as to define a cutout zone 18. This cutout zone 18 is covered with the second plastic sheet 17. These first and second plastic sheets 16, 17 are joined together along a periphery of the cutout zone 18 by means of heat-sealing technique or hot melt adhesive. In the embodiment shown, the cutout zone 18 has a vertically long rectangular shape extending from the front side of the crotch region 8 into the front waist region 6. The second plastic sheet 17 may be vertically graduated such as 1, 2, 3, ... if desired.

With the diaper arranged as described above, it is possible to detect and evaluate wetness of the core 4 due to urination through the second plastic sheet 17 covering the cutout zone 18. As a wetted range of the core 4 is enlarged every time urination is repeated, the scales 1, 2, 3, ... can indicate such a change of the wetted range. Accordingly, the diaper 1 allows occurrence of urination to be easily detected without any separately provided indicator.

Alternatively, a plurality of the cutout zones 18 may be intermittently arranged vertically of the diaper 1 without departing from the scope of the present invention. It is also possible to form the cutout zone(s) 18 so that the cutout zone(s) 18 may extend transversely rather than vertically of the diaper 1. The cutout zone(s) 18 is preferably dimensioned as small as possible in order to minimize a soiled of the soiled core 4. In the case of the embodiment shown, the cutout zone 18 is preferably dimensioned to be 7 ∼ 30 mm wide and 20 ∼ 100 mm high. When a plurality of the cutout zones 18 are intermittently arranged vertically of the diaper 1, each of these cutout zones 18 is preferably dimensioned to be 7 ∼ 20 mm wide and 7 ∼ 30 mm high.

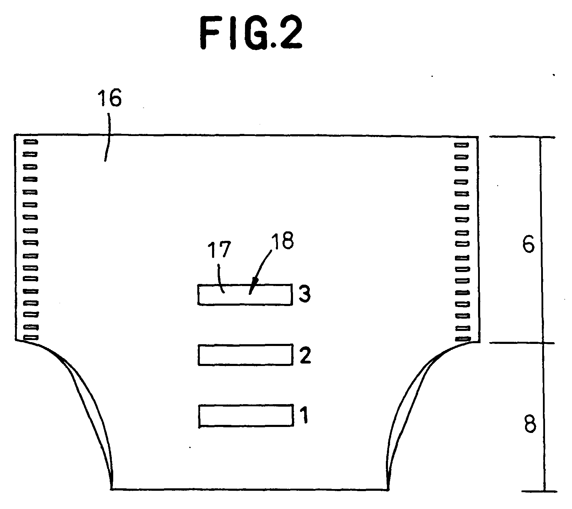

Fig. 2 is a plan view showing another embodiment of the diaper 1 according to the invention. In the crotch region 8 and, if necessary, in the front waist region 6, a plurality of transversely long cutout zones 18 may be intermittently arranged vertically of the diaper 1 wherein each of the cutout zones 18 is covered with the second plastic sheet 17.

It should be understood that the surface of the core 4 facing the cutout zone 18 may be provided with an indicator which is well known itself and adapted to become visible when wetted with urine as described in Japanese Patent Application Disclosure (Kokai) No. Hei9-140742, without departing from the scope of the present invention. Furthermore, it is also possible that the first plastic sheet 17 has a higher opacity in the vicinity of the cutout zone 18 than in the remainder.

With the novel disposable diaper disclosed herein, the backsheet is partially cut out on the front side of the crotch region and such cutout zone is covered with the transparent sheet. This unique arrangement makes it possible to detect externally of the backsheet whether the core is wetted with urine or not. Therefore, a proper timing for exchanging the soiled diaper with a fresh diaper can be reliably signaled to a user without any sanitary apprehension.

## Claims

1. A disposable diaper comprising a liquid-permeable topsheet, a liquid-impermeable backsheet and a liquid-absorbent core disposed therebetween so as to define a front waist region, a rear waist region and a crotch region extending therebetween wherein:
said backsheet covering said front and rear waist regions as well as said crotch region includes a transparent zone occupying at least a transversely middle location of said crotch region and allowing a wetness of said core to be observed and an opaque zone extending around said transparent zone and through which said core can not substantially be visible.

2. The diaper according to Claim 1, wherein said backsheet is opaque in the remainder except said transparent zone.

3. The diaper according to Claim 1, wherein said transparent zone extends from said crotch region into said front waist region.

4. The diaper according to Claim 1, wherein a surface of said core facing said transparent zone is formed with an indicator adapted to become visible when wetted with urine.

5. The diaper according to Claim 1, wherein the backsheet inclusive of said transparent zone is entirely formed of a plastic sheet.

6. The diaper according to Claim 1, wherein said transparent zone is formed on a front side of said diaper at a transversely middle location thereof.
